# EUROPÄISCHE PATENTANMELDUNG

(11) **EP 3 466 428 A1**
(43) Veröffentlichungstag der Anmeldung: **10.04.2019**
(21) Anmeldenummer: 17195524.8
(22) Anmeldetag: 09.10.2017
(51) Int. Cl.: A61K 31/517, A23K 10/30, A61P 1/12

(54) **TIERNAHRUNG MIT FEBRIFUGIN ZUR LEISTUNGSSTEIGERUNG**

(71) Anmelder: Phytobiotics Futterzusatzstoffe GmbH, 65343 Eltville (DE)
(72) Erfinder: ROTH, Dr. Hermann, 65346 Eltville (DE)
(74) Vertreter: Richardt Patentanwälte PartG mbB

(57) **Zusammenfassung**

Die Erfindung betrifft eine Tiernahrung, ein Tränkwasser oder Tiernahrungsergänzungsmittel als Leistungsförderer für Nutztiere, welche Febrifugin oder ein Febrifuginderivat enthalten.

## Beschreibung

### Technisches Gebiet

Die Erfindung bezieht sich auf Tiernahrung, Tränkwasser und einen Tiernahrungszusatz als Leistungsförderer für Nutztiere.

### Stand der Technik

Im Stand der Technik sind verschiedene Tierfutterzusatzstoffe zur Leistungsförderung bei Nutztieren beschrieben. Der Einsatz sogenannter "Leistungsförderer" ist in der heutigen Nutztierfütterung weit verbreitet. um die Wachstumsintensität der Nutztiere zu erhöhen. Noch heute (2017) werden in den allermeisten Ländern der Erde Antibiotika in niedriger Dosierung zur Leistungsstimulierung eingesetzt. dazu zählen die USA, China, Brasilien, Russland, Mexico, Australien, Japan. Daran hat auch das Verbot der Verfütterung von Antibiotika zur Leistungsverbesserung in Europa, von 2006, nichts Wesentliches geändert.

Der Einsatz von Antibiotika im Tierfutter ist mitverantwortlich für das vermehrte Auftreten von multiresistenten Keimen, z.B. multiresistente Staphylokokkus Aureus Keime (MRSA), die gegen Reserveantibiotika wie Colistin multiple Resistenzen erworben haben. Multiresistente Keime stellen eine tödliche Bedrohung für den Menschen dar, da Patienten mit geschwächtem Immunsystem, die sich mit einem Erreger infiziert haben, oftmals auf die Wirksamkeit von Antibiotika angewiesen sind um zu überleben.

Ein weiterer Nachteil von rein antimikrobiell wirkenden Leistungssteigerern ist, dass sie nicht gegen andere Faktoren, die ebenfalls das Wachstum von Nutztieren beeinträchtigen, wirken.

In dem Dokument WO 1993016602 A1 wird ein einen leistungsfördernder Tiernahrungszusatz beschrieben, der eine wirksame Menge Pflanzenmaterial der Sanguinaria Canadensis mit dem Wirkstoff Sanguinarin und anderen Isochinolinalkaloiden enthält.

### Technisches Problem und grundlegende Lösungen

Der Erfindung liegt demgemäß die Aufgabe zugrunde, weitere bzw. alternative Tiernahrungsmittel und Tiernahrungsmittelzusatzstoffe zur Leistungssteigerung bei Nutztieren zur Verfügung zu stellen.

In einem Aspekt betrifft die Erfindung eine Tiernahrung, ein Tränkwasser oder Tiernahrungsergänzungsmittel als Leistungsförderer für Nutztiere, welche Febrifugin oder ein Febrifuginderivat, enthalten. Ein Febrifuginderivat kann z.B. in einem Salz (z.B. Sulfat) von Febrifugin bestehen. Vorzugsweise wird Febrifugin verabreicht, das aus Pflanzen gewonnen wurde, z.B. in Form eines Pflanzenextrakts. Dies kann vorteilhaft sein, da somit ein weiteres, in der Herstellung kostengünstiges Mittel zur Leistungssteigerung bei Nutztieren zur Verfügung steht. So sind etwa manche alkaloidhaltigen Pflanzenextrakte im Hinblick auf die Verursachung von Antibiotikaresistenzen ebenfalls unbedenklich, doch febrifuginbasierte Leistungssteigerer haben sich in vielen Fällen als noch kostengünstiger in der Produktion erwiesen.

Febrifugin ist biologisch abbaubar und kann als Naturprodukt (Pflanzenextrakt) in vergleichsweise großen Mengen gewonnen werden. Dies entspricht auch den Wünschen des Konsumenten nach einem naturbelassenen Lebensmittel und einer nachhaltigen, umweltfreundlichen und im Hinblick auf die Entwicklung multiresistenter Erreger unbedenklichen Lebensmittelproduktion.

Somit kann in manchen Fällen auf den Einsatz von Hormonen und Anabolika, die in manchen Ländern verboten sind, verzichtet werden. bzw. der Einsatz von solchen Wachstumsförderern kann reduziert werden.

Febrifugin kommt beispielsweise in der Wurzel der Pflanze Dichroa febrifuga vor. Die Pflanze Dichroa febrifuga gehört zur Familie der Hortensiengewächse (Hydrangeaceae) und wird in China schon seit etwa 2000 Jahren als Fieber- und Malariamittel zur Behandlung von Menschen eingesetzt. Unbekannt war aber bisher die leistungsfördernde Wirkung von Febrifugin und febrifuginhaltigem Pflanzenmaterial auf Nutztiere (unabhängig davon, ob diese an Malaria leiden oder nicht). Als Anti-Malariamittel bei Nutztieren kam Febrifugin nicht in Betracht, da viele Nutztiere in Malariagebieten (z.B. Kühe und Ziegen im ländlichen Afrika) nicht an Malaria erkranken können. Experimentelle Untersuchungen ergaben jedoch, dass Febrifugin im Kontext von Nutztieren zwar als Mittel gegen Malaria kaum von Nutzen ist, dafür aber das Wachstum und die Gewichtszunahme sowie deren allgemeinen Gesundheitszustand fördert. Diese Effekte traten auch ein wenn die untersuchten Tiere weder der Malaria noch sonstigen Krankheitserregern ausgesetzt wurden. Es handelt sich hier um eine äußerst überraschende, vorteilhafte Eigenschaft dieser Substanz.

Nach Ausführungsformen wird die Tiernahrung, das Tränkwasser oder Tiernahrungsergänzungsmittel zur Vorbeugung und Bekämpfung von Darmentzündungen bei Nutztieren verwendet. Es wird davon ausgegangen, dass die Vorbeugung und/oder Bekämpfung von Darmentzündungen eine bessere Futterverwertung und damit einhergehend ein schnellerer Gewichtszuwachs ermöglicht.

Nach Ausführungsformen wird die Tiernahrung, das Tränkwasser oder das Tiernahrungsergänzungsmittel zur Vorbeugung und Bekämpfung von Durchfall bei Nutztieren verwendet. Durchfall ist eine häufige Folge von Darmentzündungen und führt aufgrund der schlechteren Resorption von Nähr- und Mineralstoffen zu einer Wachstumsverzögerung bei Nutztieren und einem allgemein verschlechterten Gesundheitszustand.

Insbesondere kann die Tiernahrung, das Tränkwasser oder Tiernahrungsergänzungsmittel zur Vorbeugung und Bekämpfung von Darmentzündungen und/oder Durchfällen, die nicht mikrobiellen Ursprungs sind (also nicht durch Bakterien, Viren, Pilze und/oder Protisten verursacht werden, sondern z.B. aufgrund von Futter oder Futterbestandteilen, das den Darmtrakt mechanisch oder chemisch reizt und zu Entzündungen führt), verwendet werden. Febrifugin und seine Derivate kann also nach Ausführungsformen der Erfindung als "Breitbandprotektiv" bzw. "Breitbandmittel" gegen (subklinische und klinische) Darmentzündungen und/oder (subklinische und klinische) Durchfallerkrankungen aller Art bei Nutztieren eingesetzt werden.

Dies kann besonders vorteilhaft sein, da pathogene Mikroorganismen nur eine von vielen Ursachen von Darmentzündungen und Durchfallerkrankungen sind, die in der Nutztierhaltung zu Leistungsverminderung führen können. So können beispielsweise auch die folgenden Umstände zu Darmentzündungen und/oder Durchfall führen, wobei die Liste nicht abschließend ist:
- haltungsbedingter Stress, insbesondere bei hoher Tierdichte auf engem Raum
- hochenergetisches Futter (insb. Jungtierfutter, Hühnerfutter)
- schwer verdauliches Futter (z.B. leiden Mastkälber in den ersten Lebenswochen unter verschiedensten entzündlichen Prozessen des Verdauungstraktes, da typischerweise schon sehr früh Rauhfutter wie Heu und geschrotetes Getreide an die Kälber verfüttert wird, obwohl deren Verdauungstrakt in diesem Alter noch auf Milchnahrung eingestellt ist. Der dadurch verursachte "Kälberdurchfall" ist für einen Großteil der Kälberverluste verantwortlich. Vor allem in den ersten beiden Lebenswochen ist das Risiko für Durchfall hoch (Neonatale Diarrhö).
- Haarballen (Bezoare) und unverdauliche Gegenstände in Magen und Darm: insbesondere Kälber in Boxenhaltung, die getrennt vom Muttertier aufwachsen, neigen dazu, an ihren Haaren und denen ihrer Artgenossen sowie an erreichbaren Einrichtungsgegenständen des Stalls zu lecken und zu saugen. Dies wird auf einen angeborenen Trieb zum Saugen zurückgeführt. Dies führt häufig zu Ansammlung von Haaren ("Haarballen") und Konglomeraten unverdaulicher Gegenstände im Magen und Darm, die zu entzündlichen Prozessen und/oder Durchfall führen können.
- mit unverdaulichem Material verunreinigtes Futter
- Futter mit hohem Anteil an ungesättigten Fettsäuren, z.B. Omega 6 Fettsäuren; insb. Futter, das zu mehr als 50%, insb. mehr als 80% aus Mais besteht, hat einen hohen Anteil an Omega 6 Fettsäuren, die als Urheber von entzündlichen Prozessen im Darm bekannt sind. Aufgrund des hohen Kaloriengehalts verbunden mit dem günstigen Preis wir Futter mit hohem Maisanteil dennoch häufig in der industriellen Tierhaltung verwendet, auch wenn dies oftmals mit chronischen Darmentzündungen der Tiere einhergeht. Aus Omega-6-Fettsäuren entstehen Botenstoffe (z.B. Prostaglandine), die deutlich entzündlicher im Stoffwechsel wirken als Botenstoffe, die aus Omega-3 entstehen. Zwar benötigen Organismen beide Arten von Fettsäuren (Omega-6-Fettsäuren beispielsweise für das Wachstum, zur Wundheilung oder zur Infektionsabwehr), sollten aber in einem für den Körper optimalen Verhältnis konsumiert werden. Bei manchen Futterarten, insbesondere bei Futter mit hohem Maisanteil, scheint ein für den Stoffwechsel von Nutztieren ungünstiges Verhältnis vorzuliegen, das Darmentzündungen fördert.

Während antibiotisch wirkende Leistungsförderer also im Wesentlichen dadurch wirken, dass sie die Keimzahl reduzieren und Keime bekämpfen, die zu Entzündungen führen, konnte von Febrifugin beobachtet werden, dass es pathogenbedingte wie auch durch sonstige, insb. nicht-pathogen bedingte Entzündungen des Darms und/oder Durchfall bekämpft bzw. diesen vorbeugt. Somit wird in vielen Fällen eine bessere Verdauung des Futters, eine bessere Futterausnutzung, eine höhere Eiweißausnutzung, und verbesserte Masttagzunahme oder Milchleistung bei gegebenem Einsatz von Futter bewirkt. Auch konnte eine ganz allgemein verbesserte Immunabwehr beobachtet werden, da der Darm für 70 % der Immunreaktion des Körpers verantwortlich ist. Gegenüber anderen natürlichen und allgemein entzündungshemmenden Substanzen wie Aspirin hat Febrifugin den Vorteil der erheblich einfacheren und dadurch kostengünstigeren Gewinnung.

Vorzugsweise enthält das Futtermittel bzw. Tränkwasser das Febrifugin oder Febrifuginderivat in physiologisch wirksamer Konzentration.

Vorzugsweise enthält das Futtermittel das Febrifugin oder Febrifuginderivat neben üblichen Futterinhaltsstoffen wie etwa Mais, Getreide, Soja oder Mischungen dieser Futterinhaltsstoffe.

Nach Ausführungsformen besteht die Tiernahrung oder Tiernahrungsergänzungsmittel zu mindestens 50%, vorzugsweise mindestens 65%, vorzugsweise 80 % seines Gewichts aus Mais. Durch Beimischung von Febrifugin kann der entzündungsfördernde Effekt der Omega-6-Fettsäure kompensiert werden. Somit ist es möglich, hochkalorisches und dabei sehr kostengünstiges Maisfutter als Tierfutter zu verwenden, ohne die mit diesem einhergehenden leistungsmindernden und entzündungsfördernden Effekte in Kauf nehmen zu müssen. Es ist also möglich, die leistungsmindernden und insb. die entzündungsfördernden Bestandteile von kostengünstigem Kraftfutter auf Maisbasis zu kompensieren.

Nach anderen Ausführungsformen besteht die Tiernahrung oder das Tiernahrungsergänzungsmittel zu mindestens 50%, insbesondere zu mindestens 65%, insbesondere zu mindestens 80 % seines Gewichts aus Getreide wie Weizen, Gerste, Roggen, Triticale und/oder Hafer. Nach manchen Ausführungsformen kann die Tiernahrung oder das Tiernahrungsergänzungsmittel Mineralstoffe und/oder Vitamine enthalten. Nach Ausführungsformen besteht die Tiernahrung oder das Tiernahrungsergänzungsmittel zu 5 bis 30 % aus Sojaschrot zur Ergänzung des Eiweißgehaltes.

Nach Ausführungsformen enthält die Tiernahrung, das Tränkwasser oder das Tiernahrungsergänzungsmittel Pflanzenmaterial einer febrifuginhaltigen Pflanze oder eines Extraktes aus einer febrifuginhaltigen Pflanze, wobei das Febrifugin oder dessen Derivat Bestandteil des Pflanzenmaterials ist. Bei der febrifuginhaltigen Pflanze kann es sich insbesondere um *Dichroa febrifuga* handeln. Insbesondere kann das Pflanzenmaterial aus Wurzelmaterial der Dichroa febrifuga bestehen oder das Wurzelmaterial beinhalten, da die Wurzeln einen besonders hohen Wirkstoffanteil enthalten.

Nach Ausführungsformen besteht die Tiernahrung oder Tiernahrungsergänzungsmittel zu mindestens 80 % seines Gewichts aus Mais, Getreide und/oder Soja.

Nach Ausführungsformen beinhaltet die Tiernahrung, das Tränkwasser oder Tiernahrungsergänzungsmittel zumindest ein Isochinolinalkaloid in einer physiologisch wirksamen Menge oder dessen Derivat.

Nach Ausführungsformen liegt das zumindest eine Isochinolinalkaloid in der Tiernahrung oder dem Tiernahrungsergänzungsmittel in Form von Pflanzenmaterial der Papaveraceae oder als Extrakt davon oder in Form der isolierten Alkaloide oder Alkaloidgemische oder synthetische Analoge vor.

Nach Ausführungsformen ist das zumindest eine Isochinolinalkaloid ein Benzophenanthridinalkaloid, vorzugsweise ausgewählt aus der Gruppe bestehend aus Sanguinarin und/oder Chelerythrin und deren Salzen.

Dies kann vorteilhaft sein, da beobachtet wurde, dass eine Kombination der Leistungssteigerer Febrifugin und z.B. Sanguinarin und/oder Chelerythrin eine synergistisch gesteigerte Leistungssteigerung bewirkt ohne dass die beiden Substanzen sich in ihrer Wirkung gegenseitig blockieren oder unerwünschte Nebenwirkungen zu beobachten waren.

Sanguinarin und verwandte Alkaloide kommen vor allem in der Sanguinaria Canadensis Pflanze, aber auch in anderen Pflanzen vor und kann z.B. in Form eines Extrakts einer sanguinarinhaltigen Pflanze dem Futtermittel bzw. Futterzusatzmittel zugegeben werden.

Nach Ausführungsformen liegt das Febrifugin bzw. Febrifuginderivat in der Tiernahrung oder dem Tränkwasser in einer Konzentration von über 50 µg/kg Tiernahrung bzw. Tränkwasser vor.

Nach Ausführungsformen liegt das Febrifugin bzw. Febrifuginderivat in der Tiernahrung oder dem Tränkwasser in einer Konzentration im Bereich von 50 µg-17mg/kg Tiernahrung bzw. Tränkwasser vor.

Nach Ausführungsformen liegt das Febrifugin bzw. Febrifuginderivat in der Tiernahrung oder dem Tränkwasser in einer Konzentration im Bereich von 1-10 mg/kg Tiernahrung bzw. Tränkwasser, vorzugsweise im Bereich von 1-4 mg/kg Tiernahrung vor.

Nach Ausführungsformen ist die Menge des zumindest einen Isochinolinalkaloids pro kg Tiernahrung bzw. pro kg Tränkwasser größer 50 µg, vorzugsweise größer 100 µg, vorzugsweise im Bereich 100 µg - 500 µg. Insbesondere liegt nach Ausführungsformen die Menge des zumindest einen Isochinolinalkaloids bei ca. 300 µg bis 1 mg pro kg Tiernahrung bzw. Tränkwasser.

Das Tiernahrungsergänzungsmittel nach einem der hier beschriebenen Ausführungsformen kann zur Erzeugung der Tiernahrung bzw. des Tränkwassers nach einem der hierin beschriebenen -Ausführungsformen verwendet werden. Das Tiernahrungsergänzungsmittel kann beispielsweise eine febrifuginhaltige (Tierfutter)vormischung oder ein febrifuginhaltiges Konzentrat sein. Beispielsweise können Dosierempfehlungen auf der Verpackung des Tiernahrungsergänzungsmittels so lauten, dass bei empfehlungsgemäßer Dosierung eine Tiernahrung bzw. ein Tränkwasser nach den hier beschriebenen Ausführungsformen beim Endabnehmer erzeugt wird.

In einem weiteren Aspekt betrifft die Erfindung ein Verfahren zur Leistungsförderung bei Nutztieren, wobei das Verfahren die Bereitstellung (Verfütterung) einer Tiernahrung bzw. des Tränkwassers nach einer der hier beschriebenen Ausführungsformen an Nutztiere beinhaltet. Die Bereitstellung kann z.B. in einem Befüllen eines Futtertrogs oder eines Futterautomaten mit febrifuginhaltigem Futter oder dem Befüllen einer Tränkanlage oder eines Wassertrogs mit febrifuginhaltigem Tränkwasser oder sonstiger Formen der Bereitstellung von erfindungsgemäßem Futter bzw. Tränkwasser an Nutztiere bestehen. Die febrifuginhaltige Tiernahrung kann fest oder flüssig sein. So wird hier z.B. auch die sogenannte "Kälbermilch" als Tiernahrung verstanden, welcher Febrifugin bzw. febrifuginhaltige Extrakte zugesetzt werden können um das Wachstum von Kälbern zu fördern, insbesondere indem die Kälber vor Darmentzündungen und Durchfall geschützt werden. Die Zusammensetzung und Darreichungsform kann in Abhängigkeit von der Nutztierart (z.B. Geflügel, Rinder, Schweine, Schafe, Ziegen, Kaninchen, etc.), dem Alter der Tiere und dem Verfütterungsort (Stall, Freiland, etc.) variieren.

Unter "Nutztieren" wird hier jegliche Tierart verstanden, die von Menschen aus kommerziellen Gesichtspunkten gehalten, gemästet und/oder vermehrt wird, insbesondere von landlebenden Säugetieren (insb. Schwein, Rind, Ziege und Schaf) und Geflügel.

Nach Ausführungsformen liegt die verabreichte Menge an Febrifugin oder Febrifuginderivat zwischen 10 µg und 3000µg, bevorzugt zwischen 20 µg und 500 µg pro kg Lebendgewicht pro Tag.

Beispielsweise kann Kälbern ein Futter, Tränkwasser oder Kälbermilch verabreicht werden, dessen Febrifugingehalt so abgestimmt ist, dass das Kalb bei normalem Nahrungskonsum ca. 50 µg - 500 µg Febrifugin pro Tag zu sich nimmt.

Nach Ausführungsformen liegt die verabreichte Menge an dem zumindest einen Isochinolinalkaloid über 1 µg pro kg Lebendgewicht pro Tag, vorzugsweise im Bereich von 10 -200 µg pro kg Lebendgewicht pro Tag.

Nach Ausführungsformen kann die Tiernahrung oder das Tränkwasser oder das Tiernahrungsergänzungsmittel ein oder mehrere Antibiotika und Arzneimittel beinhalten. Zusätzlich oder alternativ dazu kann die Tiernahrung oder das Tränkwasser oder das Tiernahrungsergänzungsmittel weitere Zusatzstoffe wie Vitamine, Spurenelemente, Aminosäuren, Kräuter, Geschmacksstoffe etc. beinhalten. Das Isochinolinalkaloid kann der Tiernahrung bzw. dem Tränkwasser oder dem Tiernahrungsergänzungsmittel in Form eines weiteren Pflanzenextrakts zugegeben werden, welcher z.B. Alkaloide wie Sanguinarin und/oder Chelerythrin enthält.

In einem weiteren Aspekt betrifft die Erfindung eine Tiernahrung, ein Tränkwasser oder Tiernahrungsergänzungsmittel zur Verwendung zur Vorbeugung und Bekämpfung von Durchfall und/oder Darmentzündungen bei Nutztieren, wobei die Tiernahrung, das Tränkwasser bzw. das Tiernahrungsergänzungsmittel Febrifugin oder ein Febrifuginderivat in physiologisch wirksamer Konzentration enthält. Der Durchfall kann ein mikrobiell-induzierter wie auch ein nicht-mikrobiell induzierter Durchfall sein. Die Darmentzündung kann eine mikrobiell-induzierte wie auch eine nicht-mikrobiell induzierte Darmentzündung sein.

Unter dem Begriff "mikrobiell-induziert" wird im Folgenden insbesondere "durch Bakterien, Viren, Pilze und/oder Protisten verursacht" verstanden. Viren gelten zwar nicht als Lebewesen, werden in der Praxis aber i.d.R. den Mikroben zugerechnet. Unabhängig von der Frage der Zuordnung von Viren unter "belebte" oder "unbelebte" Materie wird nach Ausführungsformen der Erfindung febrifuginhaltiges Futter, Tränkwasser bzw. Nahrungsergänzungsmittel zur Prophylaxe bzw. Behandlung von virus-induzierten Darmentzündungen und Durchfall erfolgreich verwendet.

Unter dem Begriff "nicht-mikrobiell-induziert" wird im Folgenden insbesondere "durch Futter oder Futterbestandteile (die den Darmtrakt mechanisch oder chemisch reizen) verursacht, haltungsbedingt oder altersbedingt" verstanden.

In einem weiteren Aspekt betrifft die Erfindung Febrifugin, ein Febrifuginderivat oder febrifuginhaltiges Pflanzenextrakt zur Herstellung einer Tiernahrung, eines Tränkwassers oder Tiernahrungsergänzungsmittel als Leistungsförderer für Nutztiere nach einer der hier beschriebenen Ausführungsformen und Darreichungsbeispiele.

### Kurzbeschreibung der Figuren

Einige Tests die die Wirksamkeit von Febrifugin zum Zwecke der Leistungssteigerung bei Nutztieren belegen sind in den Figuren 1-3 veranschaulicht. Darin zeigen:
- Figur 1: 80 Masthähnchen einer Kontrollgruppe eines Tests "Versuch I",
- Figur 2: Bilder von Darm-Sektionen der Masthähnchen von Test- und Kontroll-gruppen aus "Versuch I"
- Figur 3: ein histologischer Befund von Darm-Schnitten von Test- und Kontroll-gruppen aus "Versuch I".

### Ausführliche Beschreibung

Im Folgenden wird die Wirksamkeit von Febrifugin gegen mikrobiell wie nicht-mikrobiell induzierte Entzündungen und Durchfälle bei verschiedenen Nutztierarten anhand einiger experimenteller Daten veranschaulicht.

### Versuch I

Dieser Versuch zeigt die Wirksamkeit von Febrifugin als Leistungssteigerer sowie die Wirksamkeit von Febrifugin gegen mikrobiell induzierte Darmentzündungen und Durchfall.

Masthähnchen der Rasse "Ross" wurden ab dem Schlupf einer 42 Tage dauernden Mast unterzogen. Die Masthähnchen wurden in 7 Gruppen unterteilt. Jede Gruppe umfasste 80 Tiere, die in 8 Käfigen zu je 10 Tieren unterteilt war. Die Tiere aller Gruppen erhielten ein klassisches Basisfutter auf Mais-Soja-Basis und einem vierphasigen Fütterungsplan wie in USA und Brasilien üblich. Alle Masthähnchen wurden gemäß den Impfvorschriften der Zuchtgesellschaft geimpft und erhielten als Vorbehandlung das Antibiotikum Monensin (100 mg/kg Basisfutter). Die Tiere aller Gruppen, mit Ausnahme der Kontrollgruppe GKN, wurden am 8. Tag und am 14 Tag nach dem Schlüpftag mit Einstreumaterial aus einem Masthähnchenbestand kontaminiert, in dem zuvor Durchfallerkrankungen diverser Arten aufgetreten waren (multikomplex Durchfall mit hohem Anteil an Clostridium perfringens und E. coli Keimen). Damit wurde ein natürlicher bakterieller Stress der Tiere provoziert. Das Basisfutter einiger der Gruppen wurde mit einem leistungssteigernden Wirkstoff angereichert. Behandlungs- und Fütterungsplan der Gruppen im Einzelnen:
- **Kontrollgruppe GKN**: Basisfutter, keine Infektion
- **Kontrollgruppe GKI:** Basisfutter, Infektion an Tag 8 und 14
- **Gruppe GL-Avila:** Basisfutter + Avilamycin, Infektion an Tag 8 und 14
- **Gruppe GL-Feb-15**: Basisfutter + Febrifugin-haltiger Extrakt aus Dichroa Febrifuga (Febrifugin-Anteil am Extrakt: 6,5%); Extraktanteil von 15 mg / kg Gesamtfutter, Febrifuginanteil von ∼ 1 mg / kg Gesamtfutter, Infektion an Tag 8 und 14
- **Gruppe GL- Feb-30**: Basisfutter + Febrifugin-haltiger Extrakt aus Dichroa Febrifuga (Febrifugin-Anteil am Extrakt: 6,5%); Febrifuginanteil von ∼ 2 mg / kg Gesamtfutter, Infektion an Tag 8 und 14
- **Gruppe GL-San**: Basisfutter + Sanguinarin-haltiger Extrakt; Sanguinarinanteil von 60 mg / kg Gesamtfutter, Infektion an Tag 8 und 14
- **Gruppe GL-Oreg**: Basisfutter + Oreganoextrakt; Oregano-Extrakt-Anteil von 150 mg Extrakt/ kg Gesamtfutter, Infektion an Tag 8 und 14

| | **GKN** | **GKI** | **GL-Avila** | **GL-Feb-15** | **GL-Feb-30** | **GL-San** | **GL-Oreg** |
|---|---|---|---|---|---|---|---|
| Finales Gewicht (g) | 2460 | 2170 | 2350 | 2375 | 2340 | 2355 | 2250 |
| Relativ zu GKI | 1,13 | 1,00 | 1,08 | 1,09 | 1,08 | 1,09 | 1,04 |
| Futterverwertung | 1,80 | 2,01 | 1,84 | 1,83 | 1,85 | 1,86 | 1,90 |
| Futterverwertung relativ zu GKI | 0,90 | 1,00 | 0,92 | 0,91 | 0,92 | 0,93 | 0,95 |

Die Nullkontrolle GKN zeigt die üblichen erwarteten Leistungswerte. Alle weiteren Gruppen hatten an zwei Terminen eine mit Durchfallerregern kontaminierte Einstreu erhalten. Die Gruppe GKI, an die das Basisfutter ohne weiteren Zusatz verfüttert wurde, zeigt entsprechend reduzierte Leistungswerte um minus 13 % gegen die Kontrollgruppe GKN und einen um 10 % erhöhten Futteraufwand.

Die weiteren Versuchsgruppen zeigen alle positiv beeinflusste Leistungsparameter, wobei die beiden Gruppen mit dem Febrifugin-haltigen Futter GL-Feb-15 und GL-Feb-30 mit plus 9 % und plus 8 % im Gewicht genauso gut abschneiden wie die Gruppe GL-Avi, der das Antibiotikum Avilamycin verabreicht wurde (ebenfalls mit 8 und 9 % verbesserte Werte für Futterverwertung).

Somit zeigt sich, dass die Leistungssteigerung von Febrifugin mit der Wirksamkeit herkömmlicher Antibiotika vergleichbar ist, ohne dass die Gefahr der Förderung von multiresistenten Erregern besteht.

Dass Febrifugin nicht nur die Leistungsfähigkeit erhöht, sondern auch mikrobiellinduzierten Durchfall wirksam bekämpft bzw. reduziert, zeigen folgende ergänzende Untersuchungen, die sich auf die Feuchte des Kots (gemessen in % Wasseranteil des Kots) der oben bezeichneten Tiergruppen bezogen. Durchfall führt zu feuchterem Kot. Im Zuge der ergänzenden Untersuchungen wurde frisch abgesetzter Kot gesammelt und im Trockenschrank solange getrocknet, bis kein weiterer Gewichtsverlust durch Wasserverdunstung mehr eintrat. Die Zeit bis zur Trocknung ist ein Indikator für die ursprüngliche Feuchtigkeit des Kots. Trockenerer Kot mit geringerem Wassergehalt ist nicht nur ein Indiz, dass ein Tier gesund ist und die aufgenommene Nahrung gut verwertet, sondern hat auch für andere Tiere im gleichen Stall gesundheitlich positive Wirkung: das Stallklima ist trockener und es treten weniger Atemwegsreizungen durch Ammoniak auf, der sich in feuchter Einstreu vermehrt bilden kann.

Dass Febrifugin Entzündungen des Darms bekämpft bzw. reduziert konnte anhand morphologischer Untersuchungen festgestellt werden. Am 42. Tag wurden die Masthähnchen geschlachtet und der Darm und Teile der Darmschleimhaut präpariert. Die Darmmorphologie wurde vermessen und mikroskopisch untersucht. Um den Grad der Entzündung bzw. das Vorliegen einer Darmentzündung in der herauspräparierte Darmschleimhaut im Labor anhand ihrer Morphologie zu bewerten, wurde die Höhe der Darmzotten (Vilus Länge) und die Tiefe der Krypten vermessen. Längere Vili und tiefere Krypten zeigen eine gesündere und leistungsfähigere Darmschleimhaut-Oberfläche, von der bekannt ist, dass mit einer solchen das Futter besser verdaut werden kann. Reduzierte Werte kommen meistens von entzündlichen Prozessen. Wie anhand der Ergebnisse ersichtlich ist, wirkt Febrifugin positiv auf die Vilius-Länge und die Tiefe der Krypten und damit entzündungshemmend.

Ergänzend wurde auch der Läsionsindex des Darms der Tiere bestimmt: Durch die Zuführung alter Einstreu aus einem Bestand der an Durchfall erkrankten Masthähnchen entwickelten die Tiere der betroffenen Gruppen Symptome einer milden Clostridiose, teilweise einer milden Salmonellose und Zeichen einer Infektion mit Campylobacter. Die Clostridiose und Salmonellose führt zu Schädigung des Dünndarm-Epithels und zu entzündlichen Prozessen, die sich gut visuell diagnostizieren lassen. Basierend auf dieser visuellen Bewertung wurden Indexwerte von 1 bis 5 vergeben. Indexwert 1 steht für einen gesunden Darm, Indexwert 5 steht für einen stark geschädigten Darm, ggf. mit Blutaustritt. Die Werte dazwischen geben den Grad der Schädigung des Darms wieder.

| | **GKN** | **GKI** | **GL-Avila** | **GL-Feb-15** | **GL-Feb-30** | **GL-San** | **GL-Oreg** |
|---|---|---|---|---|---|---|---|
| Läsion | 0 | 2,5 | 1,2 | 0,8 | 0,7 | 1 | 1,8 |
| Index (g) | | | | | | | |
| Krypten Tiefe (µm) | 480 | 235 | 400 | 380 | 420 | 370 | 300 |
| Vilus Länge (µm) | 1020 | 710 | 1030 | 910 | 1020 | 1030 | 900 |
| Kot Feuchte % | 21 | 25 | 23 | 23 | 22 | 22 | 24 |

Die Morphologie, Vilus-Länge und Krypten-Tiefe war in allen Fällen der Anwendung des Extraktes der Dichroa Febrifuga verbessert und auf dem Niveau eines gesunden Darmes bei Masthähnchen (der GKN Gruppe), wo keine Infektion und keine Entzündungsprozesse stattgefunden hatten. Die Versuche belegen damit, dass Febrifugin bzw. febrifuginhaltige Pflanzenextrakte zu einer verbesserten DarmMorphologie führt, gemessen in Vilus Länge und Krypten Tiefe. Die Kotfeuchte war in den mit Febrifugin behandelten Versuchsgruppen genauso trocken wie in der Kontrollgruppe und deutlich trockener als in der mit alter Einstreu infizierten Kontrollgruppe GKI und war dem Stand der Technik (Antibiotika) überlegen oder gleichwertig. Febrifuginhaltiges Futtermittel bzw. Tränkwasser kann somit als adäquater Ersatz von Antibiotika in der Tiermast dienen.

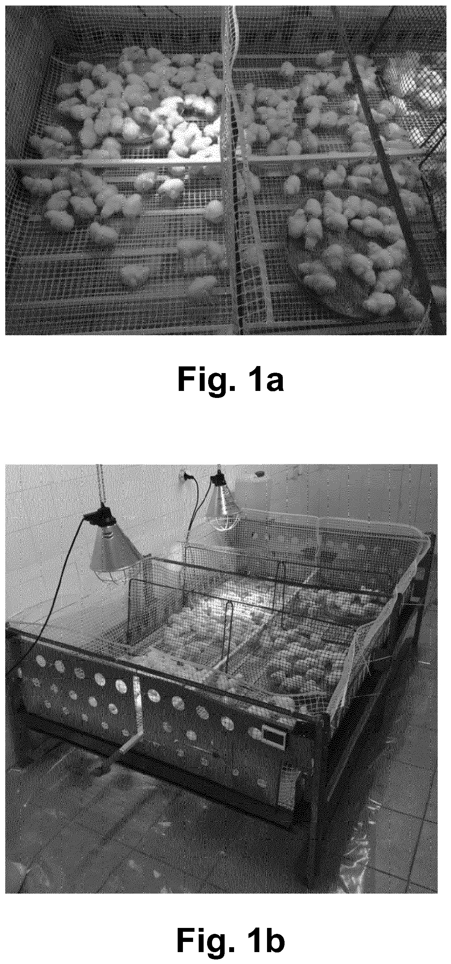

**Figur 1** zeigt 80 Masthähnchen in vier Käfigen a 20 Küken, die später auf 8 Käfige a 10 Küken verteilt und als Kontrollgruppe verwendet wurden.

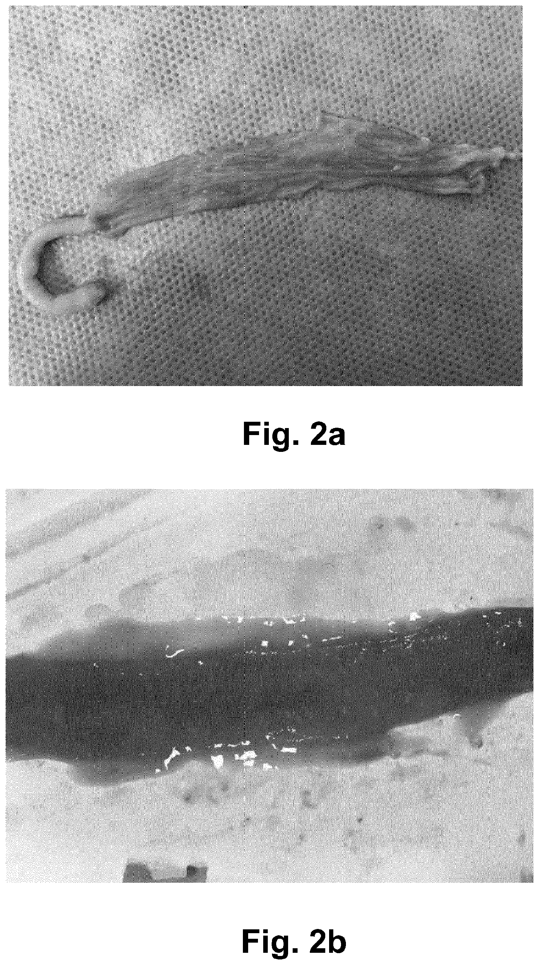

**Figur 2a** zeigt einen sezierten, gesunden Darm eines Hähnchens der Kontrollgruppe mit klar erkennbare Strukturierung (Krypten, Vili).
**Figur 2b** zeigt einen entzündeten Darm eines Hähnchens der infizierten Gruppe GKI mit unspezifischer Darmentzündung, dessen Morphologie kaum Strukturen aufweist.

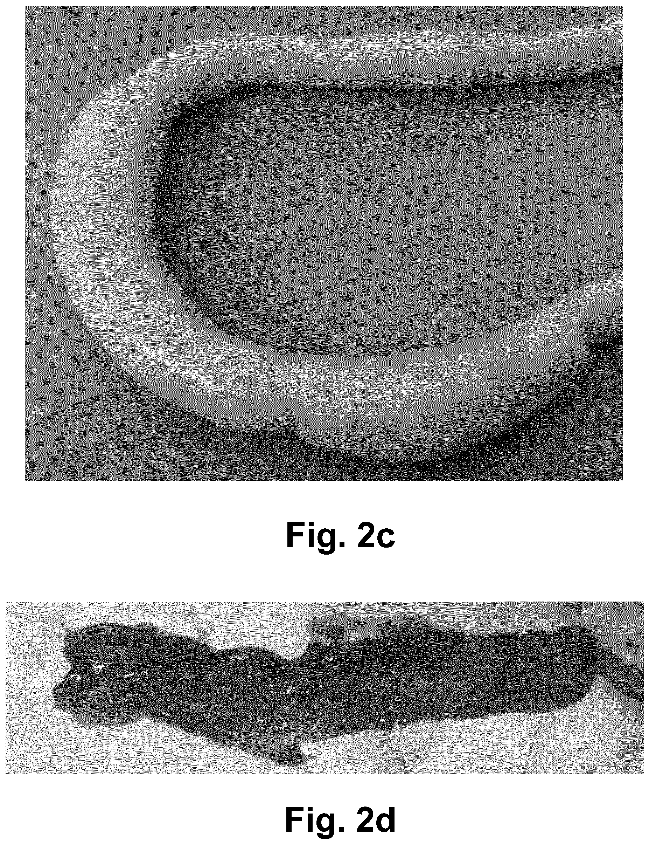

**Figur 2c** zeigt einen entzündeten und von Gärungsgasen aufgeblähten Darm eines Hähnchens der an Darmentzündung erkrankten Gruppe GKI mit unspezifischer Darmentzündung, vermutlich durch Clostridieninfektion hervorgerufen.
**Figur 2d** zeigt einen entzündeten Darm eines Hähnchens der infizierten Gruppe GKI mit unspezifischer Darmentzündung, vermutlich durch Clostridien, Salmonellen und Campylobacter hervorgerufen, die bereits in die blutige Phase übergegangen sind und dessen Morphologie kaum Strukturen aufweist.

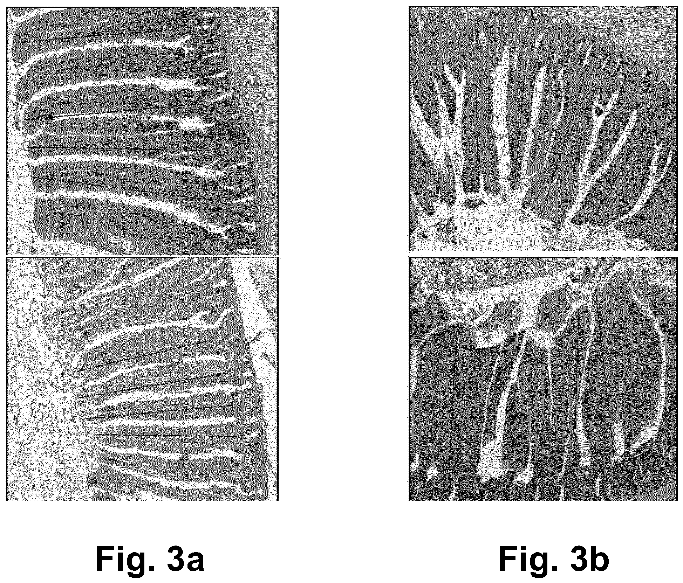

**Figur 3a** zeigt einen histologischen Befund eines Darm-Schnitts eines Hähnchens der an Darmentzündung erkrankten Gruppe GKI (unten) sowie einen entsprechenden Darm-Schnitt eines Hähnchens der Kontrollgruppe mit klar erkennbare Strukturierung (oben).
**Figur 3b** zeigt einen histologischen Befund eines Darm-Schnitts eines weiteren Hähnchens der an Darmentzündung erkrankten Gruppe GKI (unten) sowie einen entsprechenden Darm-Schnitt eines weiteren Hähnchens der Kontrollgruppe mit klar erkennbare Strukturierung (oben).

### Versuch II

Dieser Versuch zeigt die Wirksamkeit von Febrifugin als Leistungssteigerer sowie die Wirksamkeit von Febrifugin gegen nicht-mikrobiell induzierte Darmentzündungen und Durchfall.

In der Kälberhaltung treten in aller Regel etwa 10 bis 20 Tage nach der Geburt Durchfallerkrankungen auf. Ursächlich hierfür ist in den meisten Fällen nicht eine primäre bakterielle Infektion, sondern der Umstand, dass Milchkälber bereits sehr früh in ihrem Leben von teurer Milch auf Rauhfutter wie Heu und Silofutter sowie auf gepresstes industrielles Pelletfutter umgestellt werden. Zu diesem frühen Zeitpunkt ihrer Entwicklung ist der Darm noch nicht in der Lage, dieses Rauhfutter zu verwerten, sodass es zu Entzündungen kommt. Die frühe Trennung vom Muttertier verschärft dieses Problem, da Kälber einen starken Saugtrieb haben und weitgehend wahllos alle nicht fixierten Gegenstände in ihrer Umgebung, einschließlich der Haare von anderen Kälbert, aufnehmen, was zur Ausbildung von Haar- und Müllballen im Verdauungstrakt und dies wiederum zu Entzündungen und Durchfall führen kann. Es ist bekannt, dass Kälber, die bei ihren Müttern aufwachsen und dort Milch saugen können, diese Entzündungs- und Durchfall-Symptome in der Regel nicht aufweisen. Es handelt sich also klar um nicht-bakteriell induzierte Entzündungsreaktionen des Darms und nicht-bakteriell induzierten Durchfall.

Der Test wurde an insgesamt 120 frisch geborenen Kälbern durchgeführt, die in einem Bestand einer Forschungsanstalt in Deutschland gehalten wurden in welchem die Durchfallhäufigkeit bei 70 % der Kälber als normal und nicht weiter reduzierbar angesehen wurde. Es wurden zwei Gruppen gebildet: 60 Tiere der "Febrifugingruppe" GF erhielten ein febrifuginhaltiges Futter, 60 Tiere einer Kontrollgruppe GK erhielt stattdessen ein Futter, dem ein Plazebo (Kalziumcarbonat mit 1 g / Tier und Tag) beigemischt wurde.

Ein kommerzielles industrielles Mischfutter auf Mais - Getreide und Grünmehl Basis wurde mit einem Extrakt der Pflanze Dichroa Febrifuga versetzt, wobei der Extrakt einen Febrifuginanteil von 6,5% aufwies. Das febrifuginhaltige Mischfutter wurde so an die Kälber verfüttert, dass jedes Kalb 1 g des Extrakts (65 µg Febrifugin) pro Tag über einen Zeitraum vom 8. Lebenstag in der ersten Lebenswoche bis zur 27. Lebenswoche aufnahm. Es wurden jeden Tag der Tageszuwachses (g je Tag), die Durchfallhäufigkeit und die Durchfallschwere (0 = kein Durchfall, 1 = geringer Durchfall bis 3 = gelb - blutiger Kot mit hohem Wassergehalt) ermittelt.

**Ergebnisse Febrifugingruppe GF:**

| | **Tag 8** | **Tag 11** | **Tag 13** | **Tag 25** | **Tag 40** | **Woche 27** |
|---|---|---|---|---|---|---|
| #Tiere Schweregrad_1 | 0 | 6 | 1 | 0 | 0 | 0 |
| #Tiere Schweregrad_2 | 6 | 2 | 2 | 0 | 0 | 0 |
| #Tiere Schweregrad_3 | 2 | 0 | 1 | 0 | 0 | 0 |
| #Tiere Schweregrade 1, 2 und 3 | 8 | 8 | 4 | 0 | 0 | 0 |
| #Tiere Schweregrade 2 und 3 | 8 | 2 | 3 | 0 | 0 | 0 |

Im Versuch zeigt sich, dass bis zu 5 % (3/60, Tag 13) der Kälber, die Futter mit einer physiologisch wirksamen Dosis an Febrifugin erhielten, schweren Durchfall (2 und 3) entwickelten, der jedoch nach wenigen Tagen abgeklungen war. Die mittlere Durchfalldauer war 1,21 Tage über alle Tiere, in der Spitze hatten 5 % der Tiere Durchfallsymptome. Die Tageszunahme der Tiere der GF Gruppe in den ersten 3 Wochen betrug im Mittel 671 g / Tag, in der Woche 4-27 im Mittel 1430 g / Tag.

**Ergebnisse Kontrollgruppe GK (mit Plazebo):**

| | **Tag 8** | **Tag 11** | **Tag 13** | **Tag 25** | **Tag 40** | **Woche 27** |
|---|---|---|---|---|---|---|
| #Tiere Schweregrade_1 | 2 | 3 | 14 | 6 | 5 | 2 |
| #Tiere Schweregrad_2 | 7 | 8 | 10 | 6 | 2 | 1 |
| #Tiere Schweregrad_3 | 1 | 4 | 1 | 1 | 1 | 0 |
| #Tiere Schweregrade 1, 2 und 3 | 10 | 15 | 25 | 13 | 8 | 3 |
| #Tiere Schweregrade 2 und 3 | 8 | 12 | 11 | 6 | 3 | 1 |

Im Versuch zeigt sich, dass bis zu 20 % (12/60, Tag 11) der Kälber, die Futter mit dem Plazebo erhielten, schweren Durchfall (2 und 3) entwickelten, der (mit im Mittel 7 Tagen) auch deutlich länger anhielt als in der FG Gruppe. In der Spitze hatten 20 % der Tiere mittlere bis schwere Durchfallsymptome, die bis zu 10 Tage andauerten. Die Tageszunahme der Tiere der GK Gruppe in den ersten 3 Wochen betrug im Mittel 621 g / Tag, in der Woche 4-27 im Mittel 1121 g / Tag.

Der Versuch zeigt, dass Tierfutter mit einer physiologisch wirksamen Menge an Febrifugin Kälberdurchfall vorbeugen kann, der in der Regel mit Darmentzündungen einhergeht. Auch in den Fällen in welchen ein Durchfall nicht verhindert werden konnte, wurde dieser zumindest gelindert, da die Tiere mit dem Extrakt aus der Cimicifuga eine mittlere Durchfalldauer von nur 1,21 Tage hatten während die Kälber der Kontrollgruppe bis zu 7 Tage an Durchfall litten. Kälberdurchfall und die dadurch bewirke Leistungsminderung kann die Leistungsfähigkeit von Rindern bis in spätere Lebensphasen hinein negativ beeinflussen.

Der Versuch zeigt, dass febrifuginhaltiges Futter durch Verminderung klinischer und subklinischer Durchfälle und somit durch Verminderung entzündlicher Vorgänge im Darm zu einer um nahezu 26 % höheren Tageszunahme bis zur 27 Lebenswoche führte und somit die Wirtschaftlichkeit der Kälber- und Jungrindermast deutlich verbessert. Zudem ist zu berücksichtigen, dass subklinische und klinische entzündlichen Prozesse des Darms und Durchfälle einen sehr schweren Verlauf nehmen können und für einen hohen Anteil der Todesfälle während der frühen Entwicklungsphase von Rindern bei der industriellen Tiermast verantwortlich sind. Oftmals wirken sich Krankheiten in der Kindheit zudem ungünstig auf die spätere Entwicklung des Tieres zur Milchkuh oder zum Mastrind aus. Zu den im Test beobachteten direkten Effekten treten also noch Langzeiteffekte sowie Verluste durch Todesfälle ein, die durch Febrifugingabe verhindert oder reduziert werden können.

Es wird zwar bereits heute z.B. durch Verabreichung von Acetylsalicylsäure versucht, diese Entzündungsprozesse zu verhindern oder zumindest in deren Ausmaß zu reduzieren. Der Einsatz von Acetylsalicylsäure ist jedoch in vielen Ländern nicht erlaubt. Febrifuginhaltiges Futter, wozu auch febrifuginhaltige Milch gehört, oder febrifuginhaltiges Tränkwasser gemäß einer der hier beschriebenen Ausführungsformen geben dem Landwirt somit eine legale Alternative zur Kontrolle der Entzündungsvorgänge bei Kälbern an die Hand.

Verdauungsprobleme, die durch die Aufnahme von Haaren und nichtverdaulichen Gegenständen und/oder durch nicht artgerechtes oder nicht Lebenszeitaltergerechtes Futter verursacht werden, sind auch von anderen Tierarten bekannt bzw. können bei fast allen Säugetierarten, die als Nutztiere gehalten werden, beobachtet werden. Auch hier kann febrifuginhaltiges, flüssiges oder festes Futter bzw. Tränkwasser entzündliche Prozesse und Durchfall verhindern bzw. lindern und somit kurzfristig wie langfristig die Leistung der Tiere steigern.

Der für Ausführungsformen beschriebene pflanzliche Febrifuginextrakt kann beispielsweise wie nachfolgend beschrieben hergestellt werden: Die getrockneten und pulverisierten Wurzeln der Pflanze Dichroa febrifuga wurden einer zweifachen hydroalkoholischen Extraktion (70% Ethanol) unterzogen. Das Lösungsmittel wurde mittels Vakuumdestillation bei ca. 55-60°C entfernt. Dabei wurde ein konzentrierter, Extrakt gewonnen. Dieser Extrakt wurde mit Maltodextrin versetzt, der resultierende flüssige Extrakt bei ca. 80-90°C vakuumgetrocknet und anschließend gemahlen.

## Patentansprüche

1. Tiernahrung, Tränkwasser oder Tiernahrungsergänzungsmittel als Leistungsförderer für Nutztiere, das Febrifugin, oder ein Febrifuginderivat, enthält.

2. Tiernahrung, Tränkwasser oder Tiernahrungsergänzungsmittel nach Anspruch 1 zur Vorbeugung und Behandlung von mikrobiell- und/oder nicht-mikrobiell induzierten Darmentzündungen und/oder zur Vorbeugung und Behandlung von mikrobiell- und/oder nicht-mikrobiell induzierten Durchfällen bei Nutztieren.

3. Tiernahrung, Tränkwasser oder Tiernahrungsergänzungsmittel nach einem der vorigen Ansprüche, welches Pflanzenmaterial der Dichroa febrifuga oder eines Extraktes daraus, enthält, wobei das Febrifugin oder dessen Derivat Bestandteil des Pflanzenmaterials ist.

4. Tiernahrung oder Tiernahrungsergänzungsmittel nach einem der vorigen Ansprüche, welches zu mindestens 50 %, insbesondere zu mindestens 65 %, insbesondere zu mindestens 80 % seines Gewichts aus Mais besteht.

5. Tiernahrung, Tränkwasser oder Tiernahrungsergänzungsmittel nach einem der vorigen Ansprüche, wobei die Tiernahrung, das Tränkwasser oder Tiernahrungsergänzungsmittel zumindest ein Isochinolinalkaloid oder dessen Derivat beinhaltet.

6. Tiernahrung, Tränkwasser oder Tiernahrungsergänzungsmittel nach Anspruch 5, wobei das zumindest eine Isochinolinalkaloid in Form von Pflanzenmaterial der Papaveraceae oder als Extrakt davon oder in Form der isolierten Alkaloide oder Alkaloidgemische oder synthetische Analoge vorliegen.

7. Tiernahrung, Tränkwasser oder Tiernahrungsergänzungsmittel nach Anspruch 5 oder 6, wobei das zumindest eine Isochinolinalkaloid ein Benzophenanthridinalkaloid ist, vorzugsweise ausgewählt aus der Gruppe bestehend aus Sanguinarin und/oder Chelerythrin und deren Salzen.

8. Die Tiernahrung oder Tränkwasser nach einem der vorigen Ansprüche, wobei das Febrifugin in der Tiernahrung oder dem Tränkwasser in einer Konzentration von über 50µg Febrifugin/kg Tiernahrung bzw. Tränkwasser vorliegt.

9. Die Tiernahrung oder Tränkwasser nach Anspruch 8, wobei das Febrifugin in der Tiernahrung oder dem Tränkwasser in einer Konzentration im Bereich von 50µg -17 mg/kg Tiernahrung bzw. Tränkwasser vorliegt.

10. Tiernahrung oder Tränkwasser nach Anspruch 9, wobei das Febrifugin in der Tiernahrung oder dem Tränkwasser in einer Konzentration im Bereich von 1-10 mg/kg Tiernahrung bzw. Tränkwasser, vorzugsweise im Bereich von 1-4 mg/kg Tiernahrung bzw. Tränkwasser vorliegt.

11. Tiernahrung oder Tränkwasser nach Anspruch 5, 6 oder 7, wobei die Menge des zumindest einen Isochinolinalkaloids pro kg Tiernahrung oder Tränkwasser größer 50 µg, vorzugsweise größer 100 µg, vorzugsweise im Bereich 300µg - 1 mg pro kg Tiernahrung oder Tränkwasser ist.

12. Tiernahrungsergänzungsmittel nach einem der Ansprüche 1-7, wobei das Tiernahrungsergänzungsmittel eine Vormischung oder ein Konzentrat zur Erzeugung der Tiernahrung oder des Tränkwassers nach einem der Ansprüche 8-11 ist.

13. Febrifugin, Febrifuginderivat oder febrifuginhaltiges Pflanzenextrakt zur Herstellung einer Tiernahrung, eines Tränkwassers oder Tiernahrungsergänzungsmittel als Leistungsförderer für Nutztiere nach einem der Ansprüche 1-12.

14. Verfahren zur Leistungsförderung bei Nutztieren, **dadurch gekennzeichnet, dass** den Nutztieren Tiernahrung oder Tränkwasser nach einem der vorigen Ansprüche bereitgestellt wird.

15. Verfahren zur Leistungsförderung bei Nutztieren nach Anspruch 14, wobei die Leistungssteigerung durch Vorbeugung und Behandlung von mikrobiell- und/oder nicht-mikrobiell induzierten Darmentzündungen und/oder zur Vorbeugung und Behandlung von mikrobiell- und/oder nicht-mikrobiell induzierten Durchfällen bei Nutztieren bewirkt wird.

16. Verfahren nach Anspruch 14 oder 15, wobei die bereitgestellte Menge an Febrifugin oder Febrifuginderivat zwischen 10 µg und 3000 µg, bevorzugt zwischen 20 µg und 500 µg pro kg Lebendgewicht pro Tag liegt.

17. Verfahren nach Anspruch 14, 15 oder 16, wobei die verabreichte Menge an dem zumindest einen Isochinolinalkaloid über 1 µg pro kg Lebendgewicht pro Tag liegt, und vorzugsweise im Bereich von 10 µg - 200 µg pro kg Lebendgewicht pro Tag liegt.

## Geänderte Patentansprüche

### Geänderte Patentansprüche gemäss Regel 137(2) EPÜ.

1. Tiernahrung, Tränkwasser oder Tiernahrungsergänzungsmittel als Leistungsförderer für Nutztiere, das Febrifugin enthält, wobei die Tiernahrung, das Tränkwasser oder das Tiernahrungsergänzungsmittel Pflanzenmaterial der Dichroa febrifuga oder eines Extraktes daraus enthält und wobei das Febrifugin als ein Bestandteil des Pflanzenmaterials enthalten ist.

2. Tiernahrung, Tränkwasser oder Tiernahrungsergänzungsmittel nach Anspruch 1 zur Vorbeugung und Behandlung von mikrobiell- und/oder nicht-mikrobiell induzierten Darmentzündungen und/oder zur Vorbeugung und Behandlung von mikrobiell- und/oder nicht-mikrobiell induzierten Durchfällen bei Nutztieren.

3. Tiernahrung oder Tiernahrungsergänzungsmittel nach einem der vorigen Ansprüche, welches zu mindestens 50 %, insbesondere zu mindestens 65 %, insbesondere zu mindestens 80 % seines Gewichts aus Mais besteht.

4. Tiernahrung, Tränkwasser oder Tiernahrungsergänzungsmittel nach einem der vorigen Ansprüche, wobei die Tiernahrung, das Tränkwasser oder Tiernahrungsergänzungsmittel zumindest ein Isochinolinalkaloid beinhaltet.

5. Tiernahrung, Tränkwasser oder Tiernahrungsergänzungsmittel nach Anspruch 4, wobei das zumindest eine Isochinolinalkaloid in Form von Pflanzenmaterial der Papaveraceae oder als Extrakt davon oder in Form der isolierten Alkaloide oder Alkaloidgemische oder synthetische Analoge vorliegen.

6. Tiernahrung, Tränkwasser oder Tiernahrungsergänzungsmittel nach Anspruch 4 oder 5, wobei das zumindest eine Isochinolinalkaloid ein Benzophenanthridinalkaloid ist, vorzugsweise ausgewählt aus der Gruppe bestehend aus Sanguinarin und/oder Chelerythrin und deren Salzen.

7. Die Tiernahrung oder Tränkwasser nach einem der vorigen Ansprüche, wobei das Febrifugin in der Tiernahrung oder dem Tränkwasser in einer Konzentration von über 50µg Febrifugin/kg Tiernahrung bzw. Tränkwasser vorliegt.

8. Die Tiernahrung oder Tränkwasser nach Anspruch 7, wobei das Febrifugin in der Tiernahrung oder dem Tränkwasser in einer Konzentration im Bereich von 50µg -17 mg/kg Tiernahrung bzw. Tränkwasser vorliegt.

9. Tiernahrung oder Tränkwasser nach Anspruch 8, wobei das Febrifugin in der Tiernahrung oder dem Tränkwasser in einer Konzentration im Bereich von 1-10 mg/kg Tiernahrung bzw. Tränkwasser, vorzugsweise im Bereich von 1-4 mg/kg Tiernahrung bzw. Tränkwasser vorliegt.

10. Tiernahrung oder Tränkwasser nach Anspruch 4, 5 oder 6, wobei die Menge des zumindest einen Isochinolinalkaloids pro kg Tiernahrung oder Tränkwasser größer 50 µg, vorzugsweise größer 100 µg, vorzugsweise im Bereich 300µg - 1 mg pro kg Tiernahrung oder Tränkwasser ist.

11. Tiernahrungsergänzungsmittel nach einem der Ansprüche 1-6, wobei das Tiernahrungsergänzungsmittel eine Vormischung oder ein Konzentrat zur Erzeugung der Tiernahrung oder des Tränkwassers nach einem der Ansprüche 7-10 ist.

12. Febrifugin oder febrifuginhaltiges Pflanzenextrakt zur Herstellung einer Tiernahrung, eines Tränkwassers oder Tiernahrungsergänzungsmittel als Leistungsförderer für Nutztiere nach einem der Ansprüche 1-11.
